Europäisches Patentamt

European Patent Office

Office européen des brevets

(19)

(11) Publication number: **0 091 048**
A1

(12) **EUROPEAN PATENT APPLICATION**

(21) Application number: **83103036.6**

(22) Date of filing: **26.03.83**

(51) Int. Cl.³: **C 01 B 33/28**
**B 01 J 29/28**

(30) Priority: **29.03.82 US 363110**

(43) Date of publication of application:
**12.10.83 Bulletin 83/41**

(84) Designated Contracting States:
**AT BE CH DE FR GB IT LI NL SE**

(71) Applicant: **UNION CARBIDE CORPORATION**
**Old Ridgebury Road**
**Danbury Connecticut 06817(US)**

(72) Inventor: **Cannan, Thomas Richard**
**9-4 Oxford Drive**
**Valley Cottage, N.Y. 10989(US)**

(72) Inventor: **Brent, Mei-Tak Lok**
**17a Deerwood Drive**
**New City, N.Y. 10956(US)**

(72) Inventor: **Flanigen, Edith Marie**
**502 Woodland Hills Road**
**White Plains, N.Y. 10603(US)**

(74) Representative: **Eggert, Hans-Gunther, Dr.**
**Räderscheidtstrasse 1**
**D-5000 Köln 41(DE)**

(54) Zeolite LZ-132.

(57) A novel crystalline aluminosilicate composition denominated "zeolite LZ-132," or simply "LZ-132," and having uniform pore dimensions of approximately 4.3 Angstroms is prepared hydrothermally from a reaction mixture containing as essential reagents, water, soda, alumina, silica and a quaternary ammonium compound.

EP 0 091 048 A1

ZEOLITE LZ-132

S P E C I F I C A T I O N

The present invention relates in general to aluminosilicate compositions and to the method for their preparation. More particularly the invention relates to novel crystalline aluminosilicates of the zeolite type prepared hydrothermally from a reaction mixture comprising an alkali or an alkaline earth metal oxide, silica, alumina, water and methylquinuclidine ions.

The mineral zeolite levynite has been known since 1825 and has an idealized chemical composition expressed in terms of mole ratios of oxides as

$$CaO: Al_2O_3: 4 SiO_2: 6 H_2O$$

Levynite's crystal symmetry is hexagonal with unit cell parameters $a_0 = 13.32$ A and $c_0 = 22.51$ A. The X-ray powder diffraction pattern of levynite is given in "Zeolite Molecular Sieves", D.W. Breck, Wiley (1974) as set forth in Table I, below.

TABLE I

| d (A) | I | d(A) | I |
|-------|-----|-------|----|
| 10.4 | 35 | 2.725 | 6 |
| 8.19 | 65 | 2.634 | 40 |
| 7.69 | 18 | 2.593 | 6 |
| 6.72 | 18 | 2.534 | 16 |
| 5.64 | 4 | 2.453 | 2 |
| 5.19 | 30 | 2.406 | 16 |
| 4.28 | 50 | 2.303 | 10 |
| 4.10 | 100 | 2.256 | 4 |
| 3.87 | 20 | 2.234 | 14 |
| 3.84 | 6 | 2.136 | 18 |
| 3.61 | 6 | | |
| 3.49 | 16 | 2.113 | 2 |
| 3.46 | 6 | 2.072 | 6 |
| 3.35 | 14 | 2.050 | 4 |
| 3.17 | 50 | | |
| 3.10 | 20 | 1.981 | 2 |
| 2.882 | 10 | 1.960 | 6 |
| 2.861 | 10 | 1.896 | 6 |
| 2.815 | 80 | | |

D-13162

A topologically related synthetic zeolite species, denominated ZK-20, is described and claimed in U.S. Patent 3,459,676 issued August 5, 1969 to G.T. Kerr. ZK-20 is prepared hydrothermally using an organic cation as a templating agent, namely the 1-methyl-1-azonia-4 azabicyclco (2,2,2) octane ion, and has an as-synthesized chemical composition, in terms of mole ratios of oxides, of:

$0.1-0.2 \; R_2O: 0.8-0.9 \; Na_2O: Al_2O_3:$
$4-5 \; SiO_2 : 1-5 \; H_2O$

wherein "R" is the ion used as the templating agent. Its X-ray powder diffraction pattern is set forth in Table II, below:

### TABLE II

| d (A) | I | d (A) | I |
|-------|------|-------|-----|
| 14.2 | VW | 2.41 | M |
| 10.4 | M | 2.23 | W-M |
| 9.5 | W | 2.18 | VW |
| 8.2 | S | 2.14 | M |
| 7.7 | W | 2.07 | VW |
| 6.7 | M | 2.04 | W |
| 5.2 | S | 1.96 | W |
| 4.3 | S | 1.93 | W |
| 4.1 | VS | 1.90 | W |
| 3.86 | M-S | 1.88 | VW |
| 3.62 | W | 1.86 | W |
| 3.48 | W | 1.80 | M |
| 3.34 | W | 1.69 | W |
| 3.18 | S | 1.68 | M |
| 3.10 | M | 1.60 | M |
| 2.87 | M | 1.555 | M |
| 2.81 | S-VS | 1.545 | M |
| 2.64 | M | 1.435 | M |
| 2.59 | VW | 1.400 | M |
| 2.52 | W | | |

wherein with respect to the intensity values, "VW" represents Very Weak; "W" represents Weak; "M"

D-13162

represents Medium "S" represents Strong and "VS" represents Very Strong. These intensity value symbols have the same meaning when used hereinafter with respect to any X-ray powder diffraction data concerning this or other crystalline materials.

Although both of the aforesaid zeolites possess the properties of molecular sieve zeolites, i.e., crystallinity, ability to undergo ion-exchange and the ability to reversibly adsorb molecular species such as water, neither has been regarded as having significant commercial potential. This is in part because of their relatively low molar $SiO_2/Al_2O_3$ ratios, with attendant hydrophilicity, and relatively small pores. These characteristics are common to a number of commercially available zeolite species which have other and more desirable physical and/or chemical attributes. The mineral levynite, moreover, is a relatively rare species.

There has now been discovered, however, a new crystalline aluminosilicate zeolite species which is topologically related to both the mineral levynite and the synthetic ZK-20, but which differs substantially in its chemical composition from either of the previously known species. This novel zeolite, denominated LZ-132, has, prior to the extraction of any framework aluminum therefrom, a chemical composition expressed in terms of mole ratios of oxides as:

$$x \, M_{2/n}O : Al_2O_3 \; ; \; y \, SiO_2 : zH_2O$$

wherein "M is at least one cation having the valence "n", "x" has a value of from zero to 3.5 and "y" has a value of from 10 to 80, preferably 15 to 50, and "z" has a value of from 0 to 15, preferably 0 to 5, said aluminosilicate having an X-ray powder diffraction pattern containing at least the d-spacings of Table III, Table IV or Table V.

### TABLE III

| 2 theta | d(A) |
|---------|------|
| $8.8 \pm 0.3$ | $10.4 - 9.72$ |
| $11.1 \pm 0.3$ | $8.19 - 7.76$ |
| $13.6 \pm 0.3$ | $6.66 - 6.37$ |
| $17.5 \pm 0.3$ | $5.16 - 4.98$ |
| $21.2 \pm 0.3$ | $4.25 - 4.13$ |
| $22.3 \pm 0.3$ | $4.04 - 3.93$ |
| $28.7 \pm 0.3$ | $3.14 - 3.08$ |
| $32.6 \pm 0.3$ | $2.772 - 2.772_2$ |

Standard techniques were employed to obtain the data of Table III and all other X-ray data for LZ-132 appearing hereinafter. The radiation was the K doublet of copper, and a Geiger-counter spectrometer with a strip-chart pen recorder was employed. The peak heights and their positions as a function of 2 times theta, where theta is the Bragg angle, were read from the spectrometer chart and the interplanar spacing in Angstroms corresponding to the recorded lines were determined.

As synthesized, the chemical composition of LZ-132 in terms of mole ratio of oxides is

$$a\ Q_{2/n}O : bR_2O : Al_2O_3 : cSiO_2 : d\ H_2O$$

wherein "a" has a value in the range of 0.1 to 1.5, Q is an alkali metal or alkaline earth metal cation having the valence of "n", "b" has a value in the range of 0.5 to 2.5, the value of (a&b) is from 0.9 to 3.5, R represents the methylquinuclidine ion, "c"

D-13162

has a value of from 10 to 80, preferably 15 to 50, "d" has a value of from zero to about 15 depending upon the degree of dehydration and the degree to which the intracrystalline pore volume is occupied by the organic or metal cation species. The as-synthesized form of the zeolite, which can include the dehydrated (activated) form, but not the form which results from ion exchange or calcination at a temperature sufficiently high to decompose the organic constitutents, i.e., at last 400°C., but preferably 500°C. to 650°C. has a characteristic X-ray powder diffraction pattern which contains at least the d-spacings set forth in Table IV below:

### TABLE IV

| 2 theta | d(A) | Relative Intensity |
|---|---|---|
| 11.1 ± 0.3 | 8.19 - 7.76 | M |
| 17.5 ± 0.3 | 5.16 - 4.98 | VS |
| 21.2 ± 0.3 | 4.25 - 4.13 | S |
| 22.2 ± 0.3 | 4.04 - 3.93 | VS |
| 28.7 ± 0.3 | 3.14 - 3.08 | S |
| 32.6 ± 0.3 | 2.772 - 2.722 | M |

The organic cations of the as-synthesized zeolite are not considered to be ion-exchangeable in the ordinary sense due to steric considerations of the crystal structure, the organic cations themselves and the positions occupied by these cations within the crystal lattice. These large organic cations, moreover, tend to block the pore system and prevent the normal passage of the smaller metal cation species into and out of the crystal structure by the conventional ion-exchange mechanism. It is possible, however, to thermally or chemically decompose the organic cation species as

D-13162

0091048

well as any occluded organic moieties to molecular or atomic fragments small enough to beremoved through the pore systems. The organic cations associated with $AlO_4$ tetrahedra can be converted by this procedure to ammonium or hydrogen cations which are ion-exchangeable in the usual manner. Calcination of the as-synthesized zeolite in air at 650°C for one hour is sufficient to create the methylquinuclidine - free decationized form. Lower temperatures and/or shorter calcination periods are required to accomplish this result if the calcination is carried out under vacuum conditions. As will be readily apparent to those skilled in the art, the ratio of the combined organic and metallic cations to framework aluminium can appear to be substantially higher in the as-synthesized form of the zeolite than stoichiometric considerations based on conventional zeolite chemistry would permit. This phenomenon is not unique to LZ-132, however, and has generally been observed in high-silica zeolites synthesized from an organic-containing system. While the exact conditions of each organic and metal catibnic moiety of the zeolite is not known, it can be theorized that at least some of the organic species are merely occluded in the structure, and in addition entrap alkali or alkaline earth species which are not present in association with $AlO_4$ - tetrahedra. In any event there is no doubt that the topology of LZ-132 is essentially zeolitic.

It is further noted that upon calcination at temperatures sufficiently high to decompose the organic species present, the X-ray powder diffraction pattern of LZ-132 changes somewhat. The

D-13162

most notable changes appear in the intensity values for a number of d-spacings. Some shifting in line positions are also noted as well as the appearance or disappearance of one or more lines attributed to the removal of the organic species. These changes do not, however, indicate a change in the basic topology of the zeolite structure, the X-ray powder diffraction pattern of which is set forth in Table V below.

TABLE V

| 2 theta | d (A) | Relative Intensity |
|---------|-------|--------------------|
| 8.8 $\pm$ 0.3 | 10.4 - 9.72 | M |
| 11.1 $\pm$ 0.3 | 8.19 - 7.76 | VS |
| 13.6 $\pm$ 0.3 | 6.66 - 6.37 | VS |
| 17.5 $\pm$ 0.3 | 5.16 - 4.98 | M |
| 21.2 $\pm$ 0.3 | 4.25 - 4.13 | M |
| 22.3 $\pm$ 0.3 | 4.04 - 3.93 | M |

The organic-free form of the zeolite, such as prepared by the calcination of as-synthesized LZ-132 described above, either per se or after subsequent ion-exchange, has the chemical composition in the anhydrous state in terms of mole ratios of oxides

$$x\ M_{2/n}O\ :\ Al_2O_3\ :\ ySiO_2$$

wherein "M" is an ammonium, hydrogen or metal cation, "x" has a value of zero to 1.1, "y" has a value of 10 to 80, preferably 15-50, said zeolite having an X-ray powder diffraction pattern containing at least the d-spacings set forth in Table IV, supra.

Zeolite LZ-132 can be prepared hydrothermally by crystallization from a gel whose composition expressed in terms of mole ratios of oxides falls within the following ranges:

$$1.0\ Al_2O_3 : 19\text{-}80\ SiO_2 : 0.3\text{-}5.6\ R_2O :$$
$$1.0\text{-}5.0\ Q_{2/n}O : 160\text{-}600\ H_2O$$

wherein "R" represents the methylquinuclidine ion, "Q" represents an alkali metal or alkaline earth cation, preferably an alkali metal cation such as sodium, potassium or cesium, and "n" is the valence of the said metal cation. Preferably, the gel composition expressed in terms of mole-ratios of oxides should fall within the following ranges:

$$1.0\ Al_2O_3 : 15\text{-}50\ SiO_2 : 2.0\text{-}4.0$$
$$(methylquinuclidine)_2O:$$
$$1.0\text{-}4.5\ Na_2O : 350\text{-}450\ H_2O$$

In preparing the aqueous reaction mixture the conventional reactants used in zeolite synthesis are suitably employed. Alumina can be obtained from activated alumina, alpha alumina, gamma alumina, boehmite, pseudo-boehmite, alumina trihydrate, aluminum hydroxide or sodium aluminate. Silica can be obtained from precipitated silica, sodium silicate, silica sol or silica aerogel.

Advantageously, the crystallization procedure is carried out at temperatures in the range of from about 100°C to 200°C, preferably at 125°C to 150°C, the pressure being autogenous pressure, until the product is crystallized. The product is then filtered, washed add dried.

It is observed that LZ-132 is not produced as a pure product over the entire gel composition,

D-13162

temperature and time ranges indicated above. Commonly, there is also produced another novel crystalline zeolite species, denominated LZ-133, which is believed to be topologically related to LZ-132, but whose structure has not yet been determined. LZ-133 is described in detail in copending application Serial No._____ filed contemporaneously with the present application. It appears that the use of silica sols as the silica source in the reaction mixture, lower temperatures and longer crystallization periods, tend to favor the production of LZ-132, whereas precipitated amorphous silica as the silica source, coupled with higher temperatures and shorter crystallization periods, tend to favor the production of LZ-133.

The method for preparing zeolite LZ-132 is illustrated by the following examples.

Example 1

(a) Preparation of metnylquinuclidine hydroxide (MEQOH).

Initially 102.8 grams of quinuclidine (1-azabicyclo (2,2,2) octane) were dissolved in 500 grams of ethylene glycol in a one-liter 3-neck round-bottom flask fitted with a condenser, a thermometer and a mechanical glass stirring rod with an inert plastic crescent stirring blade. In a constant dropping funnel, 144.4 grams of methyl iodide ($CH_3I$) was diluted with 500 grams of ethylene glycol. The methyl iodide solution was slowly added into the quinuclidine solution with vigorous mixing. A temperature rise of 50°C was observed. After the completion of the addition of methyl iodide solution, the entire solution was mixed vigorously for another 5 hours.  Then the
solution was vacuum-distilled in a rotary
evaporator.  A total of 130 grams of dried solid
product was recovered.  A $^{13}$C NMR study of the
product indicated that methylquinuclidine iodide
(MeQI) was the principal product with trace amounts
of ethylene glycol.  (The chemical analysis results
for a similar preparation using water as a solvent
gave a carbon to nitrogen ratio of 8.5 compared to a
theoretical ratio of 8.0 (5.2 wt% N, 37.6 wt.% C on
an anhydrous basis; solution contained 69.5 wt%
MeQI).  The as-synthesized product, 128.4 grams of
methylquinuclidine iodide, was thereafter dissolved
in 128.4 grams of distilled water in a constant
dropping funnel.  Then, 129.3 grams of Ag$_2$0 was
slurried with 129.3 grams of distilled water in a
500 cc, 3-neck flask equipped with a condenser, a
mechanical stirrer and the constant dropping
funnel.  With vigorous stirring, methylquinuclidine
iodide solution was added dropwise.  After
completion of the addition, the 3-neck flask was
wrapped entirely with aluminum foil to keep the room
light out of the flask.  The vigorous stirring was
carried out overnight.  The final solution was
filtered and stored in a 500 ml polypropylene
bottle.  The final solution was chemically analyzed
and found to contain 16.0 wt% carbon, 1.86 wt%
nitrogen and 1.30 meq./gram of total alkalinity.
These data convert into a carbon to nitrogen ratio
of 10.0 (compared to a theoretical ratio of 8.0) and
a methylquinuclidine hydroxide (MeQOH) concentration
of 1.3 molar based on total alkalinity.  The higher
than theoretical C/N ratio could result from
dissolved ethylene gylcol or carbonate.

D-13162

(b)    Preparation of LZ-132

NaOH in an amount of 1.2 grams was dissolved along with 2 grams of alumina in 5 grams of distilled water with heat and stirring in a beaker.   The hot sodium aluminate solution was added to 72.9 grams of an aqueous silica sol and mixed well.   The sodium aluminate beaker was rinsed with 4 grams of distilled water and the rinse water added to the alumina-silica gel.   Then 50.5 grams of 18.6 wt% methylquinuclidine solution, prepared according to part (a) above, were added to the alumina-silica gel and mixed well.   The final gel had a molar oxide composition of 1.0 $Al_2O_3$ : 27.3 $SiO_2$ : 1.1 Na O : 4.9 methylquinuclidine hydroxide (MeQOH) : 420 $H_2O$.

The synthesis gel was then placed in a polytetrafluoroethylene container, which was in turn sealed in a stainless steel reactor, and digested at 150°C for 168 hours.   The solid reaction product was recovered by filtration, washed with water and dried at ambient temperature.   The product was identified by X-ray diffraction as zeolite LZ-132.   The chemical composition of the anhydrous product was as follows:

4.4 wt% $Al_2O_3$, 70.9 wt% $SiO_2$, 0.63 wt% $Na_2O$, 14.4 wt.% C, 2.0 wt% N, and 24.1 wt% loss on ignition at 1000°C.

The chemical composition expressed in terms of oxide mole ratios was as follows:

1.0 $Al_2O_3$ : 27.4 $SiO_2$ : 0.24 $Na_2O$ : 1.74 $(MeQ)_2O$ (The methylquinuclidine content was based on the carbon analysis).

The as-synthesized LZ-132 exhibited the following X-ray powder diffraction pattern. This pattern is essentially the same as the pattern of all other pre-calcined zeolite LZ-132 compositions which are in the mixed sodium and methylquinuclidine cation form.

TABLE VI

| d,A | I/Io |
|-----|------|
| 10.2 | 12 |
| 7.97 | 41 |
| 7.56 | 2 |
| 6.51 | 21 |
| 5.47 | 11 |
| 5.07 | 84 |
| 4.93 | 15 |
| 4.46 | 2 |
| 4.19 | 58 |
| 4.00 | 100 |
| 3.75 | 32 |
| 3.52 | 7 |
| 3.40 | 4 |
| 3.26 | 18 |
| 3.11 | 50 |
| 3.02 | 9 |
| 2.805 | 6 |
| 2.747 | 39 |
| 2.578 | 9 |
| 2.536 | 3 |
| 2.475 | 3 |
| 2.356 | 2 |
| 2.254 | 4 |
| 2.217 | 1 |
| 2.181 | 2 |
| 2.122 | 3 |
| 2.090 | 7 |
| 2.023 | 2 |
| 1.998 | 4 |
| 1.910 | 1 |
| 1.884 | 4 |
| 1.852 | 5 |
| 1.762 | 9 |
| 1.719 | 1 |
| 1.647 | 3 |

D-13162

(c)     A portion of the solid crystalline
product obtained above was calcined in air atabout
500°C for an hour.   The calcined product had an
X-ray diffraction pattern characterized by the
following data.   This pattern is essentially the
same as the patterns of all other zeolite LZ-132
compositions which have been calcined at above 400°C
for a sufficient time to be free of organic cations
and whose metal cations consist of sodium cations.

| 2θ | d A | I/Io |
|------|-------|------|
| 8.8 | 10.05 | 26 |
| 11.1 | 7.97 | 100 |
| 11.7 | 7.56 | 7 |
| 13.6 | 6.51 | 71 |
| 16.2 | 5.47 | 3 |
| 17.6 | 5.04 | 23 |
| 20.2 | 4.40 | SH |
| 21.3 | 4.17 | 27 |
| 22.4 | 3.97 | 40 |
| 23.7 | 3.75 | 14 |
| 25.4 | 3.51 | 4 |
| 26.4 | 3.38 | 3 |
| 27.6 | 3.23 | 12 |
| 28.9 | 3.09 | 4 |
| 32.1 | 2.788 | 5 |
| 32.8 | 2.730 | 20 |
| 35.0 | 2.564 | 5 |
| 35.7 | 2.515 | 3 |
| 35.5 | 2.455 | 2 |
| 38.6 | 2.332 | 1 |
| 40.0 | 2.254 | 1 |
| 41.6 | 2.171 | 1 |
| 42.8 | 2.118 | 2 |
| 43.5 | 2.080 | 2 |
| 45.0 | 2.014 | 1 |
| 45.6 | 1.989 | 1 |
| 48.2 | 1.888 | 1 |
| 48.5 | 1.877 | 2 |
| 49.6 | 1.838 | 2 |
| 50.7 | 1.801 | 3 |
| 52.1 | 1.755 | 3 |
| 53.7 | 1.707 | 1 |

(d)   Adsorption capacities were measured on the calcined product of part (c) using a standard McBain-Bakr gravimetric adsorption apparatus.  The following data were obtained on samples activated at 350°C under vaccuum for 16 hours.

|  | Kinetic Diameter, A | Pressure, torr | Temp.,°C | wt.% Absorbed |
|---|---|---|---|---|
| $O_2$ | 3.46 | 102 | -183 | 26.0 |
| $O_2$ | 3.46 | 755 | -183 | 30.1 |
| n-Butane | 4.3 | 101 | 24 | 1.4 |
| n-Butane | 4.3 | 301 | 24 | 1.9 |
| n-Butane | 4.3 | 303 | 50 | 2.7 |
| n-Hexane | 4.3 | 11.5 | 24 | 13.1 |
| n-Hexane | 4.3 | 83 | 24 | 15.0 |
| Iso-butane | 5.0 | 762 | 24 | 0.42 |
| $H_2O$ | 2.65 | 4.6 | 24 | 14.1 |
| $H_2O$ | 2.65 | 20.0 | 24 | 26.1 |

The pore size of the calcined product is 4.3A as shown by adsorption of n-hexane (kinetic diameter of 4.3A) and nil adsorption of iso-butane (kinetic diameter of 5.0A).

Examples 2-7

Using synthesis procedures similar to those detailed in Example 1, supra, six addiitional preparations of LZ-132 were made using the reagents and conditions set forth in tabular form below.  In all preparations the product was LZ-132 along with a small amount of zeolite LZ-133 as an impurity component.  LZ-133 is decribed in detail in copending application Serial No. _____ filed contemporaneously with the present application.

| Example | Synthesis Gel Composition Oxide Mole Ratio | | | | | Temp., °C | Digestion Time(hrs) | LZ-132 Product Composition, Oxide Mole Ratio | | | |
|---|---|---|---|---|---|---|---|---|---|---|---|
| | $Al_2O_3$ | $SiO_2$ | $Na_2O$ | $H_2O$ | MeQI | | | $Al_2O_3$ | $SiO_2$ | $Na_2O$ | $R_2O$ |
| 2 | 1.0 | 20.0 | 4.2 | 420 | 5.6 | 150 | 167 | 1.0 | 15.8 | 0.29 | 0.94 |
| 3 | 1.0 | 30.0 | 4.3 | 420 | 5.6 | 150 | 167 | 1.0 | 25.4 | 0.34 | 1.49 |
| 4 | 1.0 | 35.0 | 4.4 | 420 | 5.6 | 150 | 167 | 1.0 | 30.2 | 0.37 | 1.82 |
| 5 | 1.0 | 40.0 | 4.5 | 420 | 5.6 | 150 | 167 | 1.0 | 36.0 | 0.51 | 2.01 |
| 6 | 1.0 | 50.0 | 5.0 | 420 | 8.0 | 150 | 190 | 1.0 | 47.5 | 1.04 | 1.85 |
| 7 | 1.0 | 27.3 | 2.64* | 420 | 5.6 | 150 | 187 | 1.0 | 23.0 | 0.13 | 1.02 |

* Plus 1.36 $Cs_2O$

Example 8

The hydrophobic surface characteristic of LZ-132 was demonstrated by an ethanol-water shake test. A one gram sample of a stepwise calcined sample of Example 1(b) was added to a small serum bottle. The stepwise calcination procedure consisted of raising the sample temperature to 100°C., followed by a heat-up rate of 100°C/hr., with an intermittent holding time of one hour when the temperature reached 200°, 300°, 400° and 500°C, until the final temperature reached 590°C. During the heat-up time, the sample was under a nitrogen gas purge. When the temperature reached 590°C, the sample was calcined in oxygen for one hour. The 10cc of 5.0 vol. % $C_2H_5OH$ in $H_2O$ solution was introduced. The bottle was sealed and shaken thoroughly. The liquid was analyzed by gas chromatography and the result indicated a total of 14.9% ethanol removal. For comparison, acid-washed silicalite removed 23% ethanol from the same solution.

Example 9

In order to demonstrate the catalytic activity of zeolite LZ-132, a sample of Example 1(b) calcined according to Example 8 supra, was tested for the catalytic cracking of 2 mole % n-butane in helium at 500°C. The test procedure employed was the catalytic cracking of premixed 2 mole % n-butane in ahelium stream in a 1/2" O.D. quartz tube reactor over 5 grams (20-40 mesh) of the zeolite to be tested. The sample was activated in situ for 60 minutes at 500°C under 200 $cm^3$/min dry helium purge. Then the 2 mole % n-butane in helium at a flow rate of 50 $cm^3$/min was passed over the sample for 40 min. with product stream analysis being carried out at 10 minute intervals. The pseudo first order reaction rate constant ($k_\alpha$) was then calculated to determine the catalytic activity of the zeolite LZ-132. The $k_\alpha$ value for the calcined LZ-132 sample was found to be 6.7 as compared to a $k_\alpha$ value of 1.1 for $NH_4$-Y zeolite.

D-13162

What is Claimed Is:

1. Crystalline aluminosilicate zeolite composition having in its anhydrous state a chemical composition in terms of mole ratio of oxides

$$x \ M_{2/n}O : Al_2O_3 : y \ SiO_2 : zH_2O$$

wherein "M" is at least one cation having the value "n", "x" has a value of from zero to 3.5, "y" has a value of from 10 to 80 and "z" has a value of from zero to 15, said aluminosilicate having an X-ray powder diffraction pattern containing at least the d-spacings set forth in Table III, Table IV or Table V.

2. Crystalline aluminosilicate zeolite composition according to claim 1 wherein "y" has a value of from 15 to 50.

3. Crystalline aluminosilicate zeolite composition according to claim 1 which has an X-ray powder diffraction pattern containing at least the d-spacings set forth in Table IV.

4. Crystalline aluminosilicate zeolite composition according to claim 1 which has an X-ray powder diffraction pattern containing at least the d-spacings set forth in Table V.

5. Crystalline aluminosilicate zeolite composition having an as-synthesized chemical composition in terms of mole ratios of oxides:

$$a \ Q_{2/n}O : b \ R_2O : Al_2O_3 : cSiO_2 : d \ H_2O$$

wherein "a" has a value in the range of 0.1 to 1.5,

D-13162

Q is an alkali metal or alkaline earth metal cation having the valence of "n", "b" has a value in the range of 0.5 to 2.5, the value of (a+b) is from 0.9 to 3.5, R represents the methylquinuclidine ion, "c" has a value of from 10 to 80, "d" has a value of from zero to 15, said zeolite composition having an X-ray powder diffraction pattern which contains at least the d-spacings set forth in Table IV.

6.   Crystalline aluminosilicate zeolite composition according to claim 5 wherein "c" has a value of from 15 to 50.

7.   Crystalline aluminolsilicate zeolite composition according to claim 4 wherein "x" has a value of zero to 1.1 and "M" is a cation selected from the group consisting of ammonium, hydrogen or metal.

8.   Crystalline aluminosilicate composition resulting from the calcination of the composition of claim 5 at a temperature of at least 400°C up to the crystal destruction temperature thereof.

9.   Process for preparing a crystalline aluminosilicate zeolite of claim 5 which comprises forming a reaction mixture having a composition expressed in terms of mole ratios of oxides within the ranges.

1.0 $Al_2O_3$ : 10-80 $SiO_2$ : 0.35-6.6 $R_2O$ :
1.0-5.0 $Q_{2/n}O$ : 160-600 $H_2O$

wherein "R" represents the methylquinuclidine ion, "Q" represents an alkali or alkaline earth metal ion

D-13162

having the valence of "n" and maintaining said
reaction mixture under autogenous pressure at a
temperature of from about 100°C to 200°C until
crystals of the aluminosilicate are obtained.

10.   Process according to claim 9 wherein
the reaction temperature is within the range of
125°C to 150°C.

11.   Process according to claim 10 wherein
the reaction mixture composition in terms of mole
ratios of oxides is within the ranges:

$1.0 \ Al_2O_3$ : $15-50 \ SiO_2$ : $2.0-4.0 \ R_2O$ :
$1.0-4.5 \ Na_2O$ : $350-450 \ H_2O$

wherein "R" represents the methylquinuclidine ion.

12.   Process for separating the less polar
molecular species from admixture with at least one
other molecular species having a greater polarity,
said less polar species and at least one of said
greater polar species having kinetic diameters of
not greater than 4.3 A which comprises contacting
said mixture with a crystalline zeolite molecular
sieve of claim 1 or claim 5 which is at least
partially activated.

13.   Process according to claim 12 wherein
molecular species of greater polarity is water.

14.   Process for the catalytic conversion
of hydrocarbons which comprises contacting said
hydrocarbon under hydrocarbon conversion conditions
with a catalyst composition comprising a zeolite of
claim 1 or claim 5 which has been at least partially
activated.

D-13162

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| P,A | US-A-4 331 643 (M.K. RUBIN et al.) | | C 01 B 33/28<br>B 01 J 29/28 |
| D,A | US-A-3 459 676 (G.T. KERR) | | |
| A | EP-A-0 040 016 (I.C.I) | | |

TECHNICAL FIELDS
SEARCHED (Int. Cl. ³)

C 01 B 33/00
B 01 J 29/00

The present search report has been drawn up for all claims

| Place of search | Date of completion of the search | Examiner |
|---|---|---|
| BERLIN | 29-06-1983 | KESTEN W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document. but published on, or after the filing date
D : document cited in the application
L : document cited for other reasons

& : member of the same patent family. corresponding document

EPO Form 1503 03 82